# EUROPEAN PATENT SPECIFICATION

(11) **EP 3 154 922 B1**
(45) Date of publication and mention of the grant of the patent: **19.12.2018**
(21) Application number: 15805903.0
(22) Date of filing: 28.05.2015
(51) Int. Cl.: C07C 2/74, C07C 5/11, C07C 5/367, C07C 6/08, C07C 7/04, C07C 13/28, C07C 15/16

(54) **PROCESS FOR PREPARING DIALKYLBIPHENYL ISOMER MIXTURES**
VERFAHREN ZUR HERSTELLUNG VON DIALKYLBIPHENYLISOMERGEMISCHEN
PROCÉDÉ DE PRÉPARATION DE MÉLANGES D'ISOMÈRES DE DIALKYLBIPHÉNYLE

(30) Priority: 13.06.2014 US 201462012024 P; 15.07.2014 EP 14177011
(43) Date of publication of application: 19.04.2017
(73) Proprietor: ExxonMobil Chemical Patents Inc., Baytown TX 77520-2101 (US)
(72) Inventor: SALCICCIOLI, Michael, Houston, TX 77021 (US); CHEN, Tan-jen, Kingwood, TX 77345 (US); SANGAR, Neeraj, League City, TX 77573 (US); KHEIR, Ali, A., Sugar Land, TX 77498 (US); PAVLISH, Aaron, B., Humble, TX 77346 (US)
(74) Representative: ExxonMobil Chemical Europe Inc.
(86) International application number: PCT/US2015/032795
(87) International publication number: WO 2015/191289

(56) References cited:
- JP-A- H03 106 833
- JP-A- H07 173 086
- US-A- 3 928 481
- US-A- 4 123 470
- US-A1- 2005 137 437
- ANTHONIUS J. HOEFNAGEL ET AL.: 'Selective alkylation of methylbenzenes with cyclohexene catalyzed by solid acids' CATALYSIS LETTERS vol. 85, no. 1-2, 2003, pages 7 - 11, XP055109766

## Description

### FIELD OF THE INVENTION

This invention relates to a process for preparing a mixture of dialkylbiphenyl isomers, particularly a mixture of dimethylbiphenyl isomers having an increased concentration of the 3,3', 3,4', and 4,4' isomers.

### BACKGROUND OF THE INVENTION

Dimethylbiphenyl (DMBP) and other dialkylbiphenyls are useful intermediates in the production of a variety of commercially valuable products, including polyesters and plasticizers for PVC and other polymer compositions. For example, DMBP can readily be converted to an ester plasticizer by a process comprising oxidation of the DMBP to produce the corresponding mono- or dicarboxylic acid followed by esterification with a long chain alcohol. However, for certain uses, it is important to reduce the level of 2,X' DMBP (where X' is 2', 3' and 4') isomers in the product since, for example, diphenate esters having substitution on the 2-carbons tend to be too volatile for use as plasticizers.

As disclosed in US 2014/0275609 A1 and US 2014/0275605 A1, dimethylbiphenyl may be produced by hydroalkylation of toluene followed by dehydrogenation of the resulting (methylcyclohexyl)toluene (MCHT). However, even using a selective molecular sieve catalyst for the hydroalkylation step, this process tends to yield a mixture of all six DMBP isomers, namely 2,2', 2,3' 2,4', 3,3', 3,4' and 4,4' DMBP, in which the 2,X' DMBP isomer content may be 20% by weight or more of the total DMBP product. Moreover, 2,2', 2,3' and 2,4' DMBP cannot be completely separated from unreacted MCHT by distillation due to an overlap in their vapor-liquid equilibrium properties. There is, therefore, interest in developing a process for converting 2,X' DMBP isomers into 3,3', 3,4' and 4,4' DMBP and especially a process which effects such a conversion with minimal loss of any unconverted MCHT which may be present in the DMBP feed.

The production of biphenylesters is based on the initial steps of aromatic hydroalkylation (see US 2014/0275606 A1, US 2014/0275609 A1 and 2014/0275605 A1), followed by dehydrogenation to produce biphenyls or alkylbiphenyls (see US 2014/0212666 A1, US 2014/0275609 A1, and US 2014/0275607 A1). US patent 3,928,481 relates to the preparation of polyphenyls by selective dehydrogenation of cyclohexyl benzenes. It disclosed the dehydrogenation of products of hydroalkylation to form desired aromatic hydrocarbons.

### SUMMARY OF THE INVENTION

According to the present invention, it has now been found that the amount of 2,X' DMBP isomers in a DMBP isomer mixture can be reduced by a combination of hydrogenation of the DMBP back to MCHT, followed by transalkylation of the MCHT with toluene and then dehydrogenation of the transalkylation product back to DMBP. In particular, it is found that steric issues favor the transalkylation of 1-methyl-2-(X-methylcyclohexyl)benzene (where X = 2, 3 or 4) with toluene to produce 1-methyl-Y-(X-methylcyclohexyl)benzene (where Y= 3 or 4 and X is the same position as the feed).

In one aspect, the invention resides in a process for producing dialkylbiphenyl compounds, the process comprising:
(a1) providing a feed comprising substituted cyclohexylbenzene isomers having the formula (I): 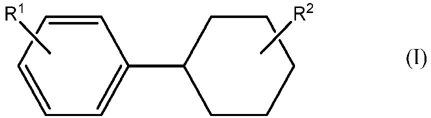 wherein each of R¹ and R² is an alkyl group and wherein the feed comprises m % by weight of isomers in which R¹ is in the 2-position, based on the total weight of substituted cyclohexylbenzene isomers in the feed;
(a2) reacting the feed under transalkylation conditions with a compound of formula (II): 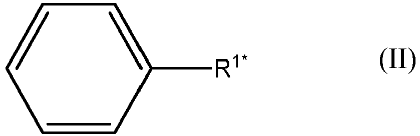
   wherein R^{1*} is an alkyl group,
   to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
(a3) dehydrogenating at least part of the transalkylation product under conditions effective to convert at least part of the substituted cyclohexylbenzene isomers in the transalkylation product to dialkylbiphenyl compounds.

In a further aspect, the invention resides in a process for reducing the amount of 2,X' dialkylbiphenyl isomers (where X' is 2', 3' and/or 4') in a first mixture containing the same, the process comprising:
(b1) hydrogenating the first mixture under conditions effective to produce a hydrogenation product comprising substituted cyclohexylbenzene isomers having the formula (I): 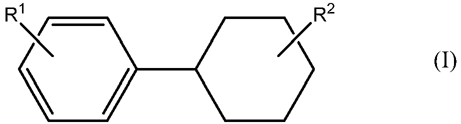 wherein each of R¹ and R² is an alkyl group and wherein the hydrogenation product comprises m % by weight of isomers in which R¹ is in the 2-position, based on the total weight of substituted cyclohexylbenzene isomers in the hydrogenation product;
(b2) reacting the hydrogenation product under transalkylation conditions with a compound of formula (II): 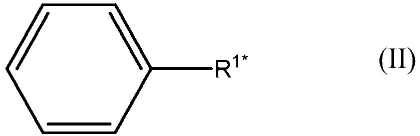
   where R^{1*} is an alkyl group,
   to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
(b3) dehydrogenating at least part of the substituted cyclohexylbenzene isomers in the transalkylation product to produce a product mixture containing less 2,3' and 2,4' dialkylbiphenyl isomers than the first mixture.

In yet a further aspect, the invention resides in a process for producing dimethylbiphenyl compounds, the process comprising:
(c1) contacting toluene with hydrogen under hydroalkylation conditions effective to produce a hydroalkylation product comprising (methylcyclohexyl)toluenes;
(c2) dehydrogenating at least part of the hydroalkylation product under conditions effective to produce a first dehydrogenation product comprising dimethylbiphenyl isomers and unreacted (methylcyclohexyl)toluenes;
(c3) separating the first dehydrogenation product into a first fraction comprising one or more 3,3', 3,4' and 4,4' dimethylbiphenyl isomers and a second fraction comprising one or more 2,X'-dimethylbiphenyl isomers (where X' is 2, 3 or 4) and at least part of the unreacted (methylcyclohexyl)toluenes;
(c4) hydrogenating at least part of the dimethylbiphenyl isomers in the second fraction to the corresponding (methylcyclohexyl)toluene isomers and produce a hydrogenation effluent;
(c5) reacting at least part of the hydrogenation effluent from (c4) with toluene under transalkylation conditions effective to produce a transalkylation product containing less material represented by Formula (X) than the hydrogenation effluent, where Formula (X) is 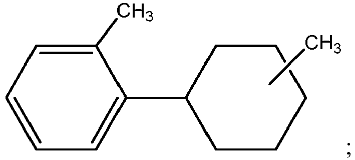 and
(c6) dehydrogenating at least part of the transalkylation product to produce a second dehydrogenation product comprising less 2,X'-dimethylbiphenyl isomers than the first dehydrogenation product.

### BRIEF DESCRIPTION OF THE DRAWINGS

Figure 1 is a flow diagram of a process for producing a mixture of dimethylbiphenyl isomers according to one embodiment of the invention.
Figure 2 is a flow diagram of a process for producing a mixture of dimethylbiphenyl isomers according to a further embodiment of the invention.
Figure 3 is a graph of C₁₃ yield as a function of temperature at different pressures in the translkylation of cyclohexylbenzene with toluene according to Example 1.
Figure 4 is bar graph showing the normalized weight percent of major reactor effluent liquid species of carbon number 12 or higher for the sequential hydrogenation and transalkylation process of Example 2.

### DETAILED DESCRIPTION OF THE EMBODIMENTS

Described herein is a process for producing dialkylbiphenyl compounds useful as precursors in the manufacture of polyesters and biphenyl ester plasticizers. In particular, the present process provides a route for the production of a mixture of dialkylbiphenyl isomers in which the amount of the 3,3', 3,4' and 4,4'-dialkylbiphenyl isomerizers is maximized and the amount of the 2,X' dialkylbiphenyl isomers (where X' is 2', 3' and/or 4') is minimized.

By way of illustration, the 3,3', 3,4' and 4,4'-isomers of dimethylbiphenyl are shown below in formulas (III) to (V) respectively, whereas the 2,2', 2,3' and 2,4'-isomers are shown in formulas (VI) to (VIII) respectively:

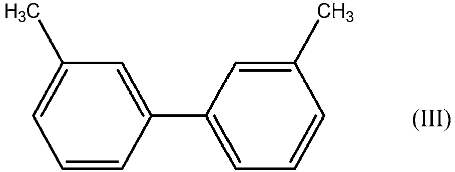

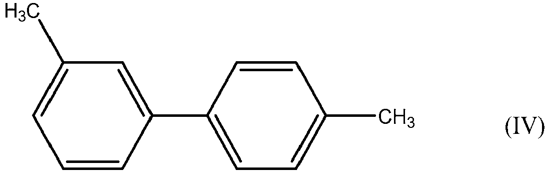

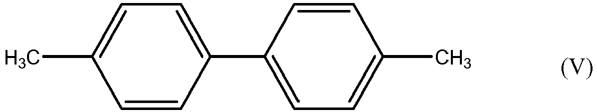

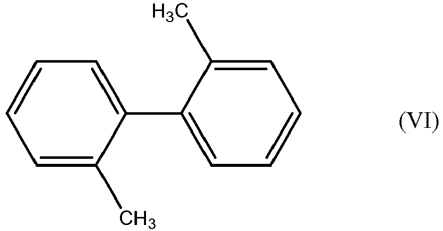

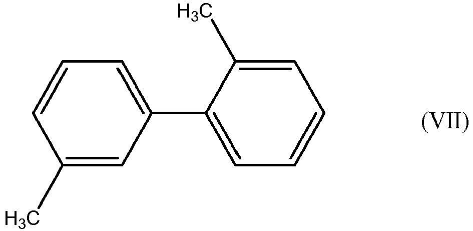

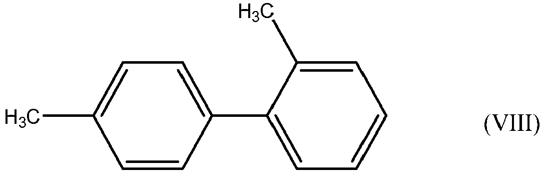

In its simplest form, the present process starts with a feed comprising a first mixture of substituted cyclohexylbenzene isomers having the formula (I): 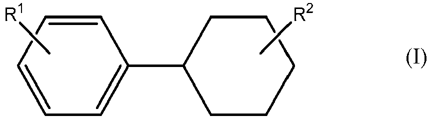 wherein each of R¹ and R² is an alkyl group and wherein the feed comprises m % by weight of isomers in which R¹ is in the 2-position, based on the total weight of substituted cyclohexylbenzene isomers in the feed. The number of carbon atoms in each alkyl group can be the same or different, but in most embodiments will be the same and will be less than 10, such as from 1 to 5. Normal alkyl groups are preferred and most preferably each of R¹ and R² is a methyl group.

In the present process, the feed comprising the mixture of substituted cyclohexylbenzene isomers having the formula (I) is initially transalkylated with a compound of formula (II): 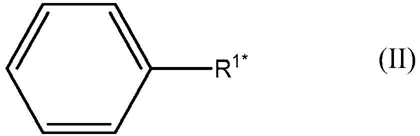 where R^{1*} is an alkyl group (preferably having 1 to 10 carbon atoms, preferably 1 to 5 carbon atoms, preferably R^{1*} is methyl), to produce a transalkylation product comprising a second mixture of substituted cyclohexylbenzene isomers having the formula (I) and including n % by weight of isomers in which R¹ is in the 2-position, based on the total weight of substituted cyclohexylbenzene isomers in the transalkylation product. As a result of steric issues, it is found that the transalkylation favors the production of the cyclohexylbenzene isomers in which the R¹ group is in the 3-position or the 4-position so that n<m. The discrete values for m and n can vary widely depending on the process used to produce the first mixture substituted cyclohexylbenzene isomers and the conditions and catalyst employed in the transalkylation step. However, in certain embodiments, m is at least 1%, such as at least 15%, for example, from 15% to 30% and n is less than 99%, such as less than 60%, for example from 1% to 15%. In an alternate embodiment, m is at least 5 wt% and n is less than 75 wt%.

The transalkylation reaction can be conducted over a wide range of conditions but in most embodiments is effected at a temperature from 75°C to 250°C, such as from 100°C to 200°C, for example 125°C to 160°C and a pressure from 100 to 3550 kPa-absolute, such as from 1000 to 1500 kPa-absolute. The reaction is normally conducted in the presence of a solid acid catalyst, such as a molecular sieve and in particular a molecular sieve having a large pore molecular sieve having a Constraint Index (as defined in US 4,016,218) less than 2. Suitable large pore molecular sieves include zeolite beta, zeolite Y, Ultrastable Y (USY), Dealuminized Y (Deal Y), mordenite, ZSM-3, ZSM-4, ZSM-18, ZSM-20, and mixtures thereof. Other suitable molecular sieves include molecular sieves of the MCM-22 family, including MCM-22 (described in US 4,954,325), PSH-3 (described in US 4,439,409), SSZ-25 (described in US 4,826,667), ERB-1 (described in EP 0293032), ITQ-1 (described in US 6,077,498), ITQ-2 (described in WO 97/17290), MCM-36 (described in US 5,250,277), MCM-49 (described in US 5,236,575), MCM-56 (described in US 5,362,697) and mixtures thereof.

According to the present process, at least part of the transalkylation product is then catalytically dehydrogenated to produce the desired mixture of dialkylbiphenyl isomers. The catalyst employed in the dehydrogenation process is not critical but, in some embodiments, comprises (i) an element or compound thereof from Group 10 of the Periodic Table of Elements, for example platinum, and (ii) tin or a compound of tin, both mounted on a refractory support, such as silica, alumina or carbon nanotubes. Suitable catalysts comprise a Group 10 element in an amount from 0.1 to 5 wt% of the catalyst and tin in an amount from 0.05 to 2.5 wt% of the catalyst. The dehydrogenation is conveniently conducted at a temperature from about 200°C to about 600°C and a pressure from about 100 kPa-absolute to about 3550 kPa-absolute (atmospheric to about 500 psig).

The amount of 2,X' isomers in the resultant mixture of dialkylbiphenyl isomers will of course depend on the value of n in the substituted cyclohexylbenzene isomers produced by the transalkylation step and the level of conversion of each MCHT isomer in the dehydrogenation step. However, in representative embodiments, the dehydrogenation product contains less than 30%, for example from 10 to 25% of 2,X'-dialkylbiphenyl isomers based on the total weight of dialkylbiphenyl compounds in the dehydrogenation product.

Although any known method can be used to produce the mixture of substituted cyclohexylbenzene isomers having the formula (I) employed as the feed in the above-described process, in one embodiment the feed is produced by hydrogenation of a precursor mixture of dialkylbiphenyl isomers. In this case, the overall process can be summarized as follows:
(i) Hydrogenation of a precursor dialkylbiphenyl isomer mixture to produce a first mixture of substituted cyclohexylbenzene isomers having the formula (I).
(ii) Transalkylation of the product of (i) with a compound of formula (II) to produce a second mixture of substituted cyclohexylbenzene isomers having a different isomer distribution than the first mixture.
(iii) Dehydrogenation of the product of (ii) to produce a further dialkylbiphenyl isomer mixture having a different isomer distribution than the precursor mixture.

In the above embodiment, the present process effectively provides a three-step method of isomerizing a mixture of dialkylbiphenyl isomers so as to reduce the level of 2,X' isomers in the mixture.

In one preferred embodiment, where each of R¹ and R² in formula (I) is a methyl group, the present process forms part of the product recovery system of an integrated process for selectively converting toluene to 3,3', 3,4' and 4,4' dimethylbiphenyl. In such a process, toluene is initially converted to (methylcyclohexyl)toluenes over a hydroalkylation catalyst according to the following reaction:

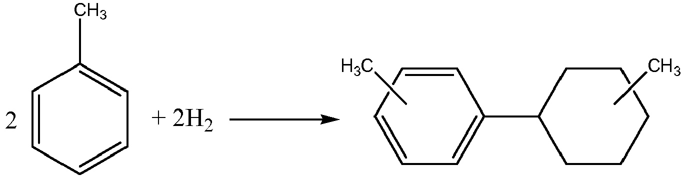

The catalyst employed in the hydroalkylation reaction is a bifunctional catalyst comprising a hydrogenation component and a solid acid alkylation component, typically a molecular sieve. The catalyst may also include a binder such as clay, alumina, silica and/or metal oxides. The latter may be either naturally occurring or in the form of gelatinous precipitates or gels including mixtures of silica and metal oxides. Naturally occurring clays which can be used as a binder include those of the montmorillonite and kaolin families, which families include the subbentonites and the kaolins commonly known as Dixie, McNamee, Georgia and Florida clays or others in which the main mineral constituent is halloysite, kaolinite, dickite, nacrite or anauxite. Such clays can be used in the raw state as originally mined or initially subjected to calcination, acid treatment or chemical modification. Suitable metal oxide binders include silica, alumina, zirconia, titania, silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia.

Any known hydrogenation metal or compound thereof can be employed as the hydrogenation component of the catalyst, although suitable metals include palladium, ruthenium, nickel, zinc, tin, and cobalt, with palladium being particularly advantageous. In certain embodiments, the amount of hydrogenation metal present in the catalyst is between about 0.05 and about 10 wt%, such as between about 0.1 and about 5 wt%, of the catalyst. In an embodiment, the hydrogenation catalyst (used in step (b1)) is a transition metal, preferably a Group 3, 4, 5, 6, 7, 8, 9, 10, 11, or 12 metal, preferably Ru, Rh, Pd, Ag, Os, Ir, Pt, A, or preferably Pt or Pd.

In one embodiment, the solid acid alkylation component comprises a large pore molecular sieve having a Constraint Index (as defined in US 4,016,218) less than 2. Suitable large pore molecular sieves include zeolite beta, zeolite Y, Ultrastable Y (USY), Dealuminized Y (Deal Y), mordenite, ZSM-3, ZSM-4, ZSM-18, and ZSM-20. Zeolite ZSM-4 is described in US 4,021,447. Zeolite ZSM-20 is described in US 3,972,983. Zeolite Beta is described in US 3,308,069, and Re. 28,341. Low sodium Ultrastable Y molecular sieve (USY) is described in US 3,293,192 and 3,449,070. Dealuminized Y zeolite (Deal Y) may be prepared by the method found in US 3,442,795. Zeolite UHP-Y is described in US 4,401,556. Mordenite is a naturally occurring material but is also available in synthetic forms, such as TEA-mordenite (i.e., synthetic mordenite prepared from a reaction mixture comprising a tetraethylammonium directing agent). TEA-mordenite is disclosed in US 3,766,093 and 3,894,104.

In another more preferred embodiment, the solid acid alkylation component comprises a molecular sieve of the MCM-22 family. The term "MCM-22 family material" (or "material of the MCM-22 family" or "molecular sieve of the MCM-22 family"), as used herein, includes one or more of:
- molecular sieves made from a common first degree crystalline building block unit cell, which unit cell has the MWW framework topology. (A unit cell is a spatial arrangement of atoms which if tiled in three-dimensional space describes the crystal structure. Such crystal structures are discussed in the "Atlas of Zeolite Framework Types", Fifth edition, 2001);
- molecular sieves made from a common second degree building block, being a 2-dimensional tiling of such MWW framework topology unit cells, forming a monolayer of one unit cell thickness, preferably one c-unit cell thickness;
- molecular sieves made from common second degree building blocks, being layers of one or more than one unit cell thickness, wherein the layer of more than one unit cell thickness is made from stacking, packing, or binding at least two monolayers of one unit cell thickness. The stacking of such second degree building blocks can be in a regular fashion, an irregular fashion, a random fashion, or any combination thereof; and
- molecular sieves made by any regular or random 2-dimensional or 3-dimensional combination of unit cells having the MWW framework topology.

Molecular sieves of MCM-22 family generally have an X-ray diffraction pattern including d-spacing maxima at 12.4±0.25, 6.9±0.15, 3.57±0.07 and 3.42±0.07 Angstrom. The X-ray diffraction data used to characterize the material are obtained by standard techniques using the K-alpha doublet of copper as the incident radiation and a diffractometer equipped with a scintillation counter and associated computer as the collection system. Molecular sieves of MCM-22 family include MCM-22 (described in US 4,954,325), PSH-3 (described in US 4,439,409), SSZ-25 (described in US 4,826,667), ERB-1 (described in EP 0293032), ITQ-1 (described in US 6,077,498), ITQ-2 (described in WO 97/17290), MCM-36 (described in US 5,250,277), MCM-49 (described in US 5,236,575), MCM-56 (described in US 5,362,697) and mixtures thereof.

In addition to the toluene, benzene and/or xylene and hydrogen, a diluent, which is substantially inert under hydroalkylation conditions, may be included in the feed to the hydroalkylation reaction. In certain embodiments, the diluent is a hydrocarbon, in which the desired cycloalkylaromatic product is soluble, such as a straight chain paraffinic hydrocarbon, a branched chain paraffinic hydrocarbon, and/or a cyclic paraffinic hydrocarbon. Examples of suitable diluents are decane and cyclohexane. Although the amount of diluent is not narrowly defined, desirably the diluent is added in an amount such that the weight ratio of the diluent to the aromatic compound is at least 1:100; for example at least 1:10, but no more than 10:1, desirably no more than 4:1.

The hydroalkylation reaction can be conducted in a wide range of reactor configurations including fixed bed, slurry reactors, and/or catalytic distillation towers. In addition, the hydroalkylation reaction can be conducted in a single reaction zone or in a plurality of reaction zones, in which at least the hydrogen is introduced to the reaction in stages. Suitable reaction temperatures are between about 100°C and about 400°C, such as between about 125°C and about 250°C, while suitable reaction pressures are between about 100 and about 7,000 kPa, such as between about 500 and about 5,000 kPa. The molar ratio of hydrogen to aromatic feed is typically from about 0.15:1 to about 15:1.

In the present process, it is found that MCM-22 family molecular sieves are particularly active and stable catalysts for the hydroalkylation of toluene or xylene. In addition, catalysts containing MCM-22 family molecular sieves exhibit improved selectivity to the 3,3'-dimethyl, the 3,4'-dimethyl, the 4,3'-dimethyl and the 4,4'-dimethyl isomers in the hydroalkylation product, while at the same time reducing the formation of fully saturated and heavy by-products. For example, using an MCM-22 family molecular sieve with a toluene feed, it is found that the hydroalkylation reaction product may comprise:
- at least 60 wt%, such as at least 70 wt%, for example at least 80 wt% of the 3,3', 3,4', 4,3' and 4,4'-isomers of (methylcyclohexyl)toluene based on the total weight of all the (methylcyclohexyl)toluene isomers;
- less than 40 wt%, such as less than 30 wt%, for example from 15 to 25 wt% of the 2,2', 2,3', and 2,4'-isomers of (methylcyclohexyl)toluene based on the total weight of all the (methylcyclohexyl)toluene isomers;
- less than 30 wt% of methylcyclohexane and less than 2% of dimethylbicyclohexane compounds; and
- less than 1 wt% of compounds containing in excess of 14 carbon atoms.

The hydroalkylation reaction product may also contain significant amounts of residual toluene, for example, up to 50 wt%, such as up to 90 wt%, typically from 60 to 80 wt% of residual toluene based on the total weight of the hydroalkylation reaction product. Thus, the major components of the hydroalkylation reaction effluent are (methylcyclohexyl)toluenes, residual toluene and fully saturated single ring by-product (methylcyclohexane). The residual toluene and light by-products can readily be removed from the reaction effluent by, for example, distillation. The residual toluene can then be recycled to the hydroalkylation reactor, while the saturated by-products can be dehydrogenated to produce additional recyclable feed.

The remainder of the hydroalkylation reaction effluent, composed mainly of (methylcyclohexyl)toluenes, is then dehydrogenated to convert the (methylcyclohexyl)toluenes to the corresponding methyl-substituted biphenyl compounds. The dehydrogenation is conveniently conducted at a temperature from about 200°C to about 600°C and a pressure from about 100 kPa to about 3550 kPa (atmospheric to about 500 psig) in the presence of dehydrogenation catalyst. A suitable dehydrogenation catalyst comprises one or more elements or compounds thereof selected from Group 10 of the Periodic Table of Elements, for example platinum, on a support, such as silica, alumina or carbon nanotubes. In one embodiment, the Group 10 element is present in amounts from 0.1 to 5 wt% of the catalyst. In some cases, the dehydrogenation catalyst may also include tin or a tin compound to improve the selectivity to the desired methyl-substituted biphenyl product. In one embodiment, the tin is present in amounts from 0.05 to 2.5 wt% of the catalyst.

Particularly using an MCM-22 family-based catalyst for the upstream hydroalkylation reaction, the product of the dehydrogenation step comprises dimethylbiphenyl compounds in which the concentration of the 3,3'-, 3,4'- and 4,4' isomers is at least 50 wt%, such as at least 60 wt%, for example at least 70 wt% based on the total weight of dimethylbiphenyl compounds. Typically, the concentration of the 2,X'-dimethylbiphenyl isomers in the dehydrogenation product is less than 50 wt%, such as less than 30 wt%, for example from 5 to 25 wt% based on the total weight of dimethylbiphenyl compounds.

By virtue of their higher boiling point, the 3,3'-, 3,4'- and 4,4'-dimethylbiphenyl isomers can readily be separated from the hydrogenation product by distillation, but 2,2', 2,3' and 2,4' isomers cannot be completely separated from residual (methylcyclohexyl)toluenes by distillation due to an overlap in their vapor-liquid equilibrium properties. In the present process, this problem is alleviated in that, after recovery of at least some of the 3,3'-, 3,4'- and 4,4' isomers, part or all of the remaining dehydrogenation product is subjected to the three-step "isomerization" process, described above, namely hydrogenation, followed by transalkylation followed by dehydrogenation, to convert at least some of 2,X' isomers to their 3,3'-, 3,4'- and 4,4'-counterparts. This process can be repeated effectively to convert all the 2,X'-dimethylbiphenyl compounds to their more desirable 3,3'-, 3,4'- and 4,4' isomers. In addition, it is found that the loss in dimethylbiphenyl yield through conversion to unwanted by-products during the three-step "isomerization" process is very low thereby enhancing the overall selectivity and yield of the integrated process for converting toluene to 3,3'-, 3,4'- and 4,4'-dimethylbiphenyl compounds.

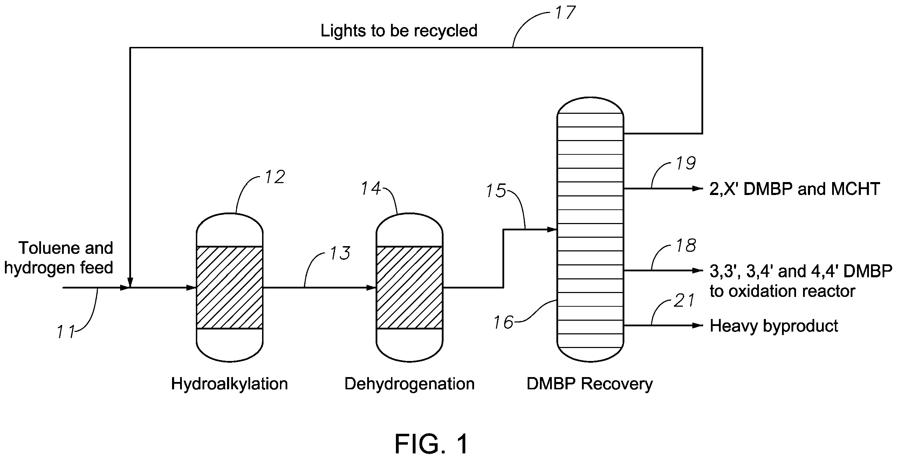

One embodiment of such an integrated process is illustrated in Figure 1, in which toluene and hydrogen are fed by a single line 11 or, if preferred by separate lines (not shown), to a hydroalkylation unit 12. The hydroalkylation unit 12 contains a bed of a bifunctional catalyst which comprises a hydrogenation component and a solid acid alkylation component and which converts the toluene to (methylcyclohexyl)toluene (MCHT). The effluent from the hydroalkylation unit 12, composed mainly of MCHT and unreacted toluene, is then fed via line 13 to a dehydrogenation unit 14 where the MCHT is dehydrogenated to produce dimethyldiphenyl (DMBP) and hydrogen. If preferred, distillation or other separation techniques can be implemented to concentrate the MCHT or remove hydroalkylation byproducts prior to the dehydrogenation step (stream 13).

The effluent from the dehydrogenation unit 14 is then supplied by line 15 to a DMBP recovery system 16, including one or more distillation columns, where hydrogen and unreacted toluene are removed as overhead and recycled via line 17 to the hydroalkylation unit 12. Also removed from the dehydrogenation effluent by DMBP recovery system 16 are a first intermediate stream rich in 3,3'-, 3,4'- and 4,4'-DMBP isomers, which is recovered as product via line 18, and a second intermediate stream rich in 2,X'-DMBP isomers and residual MCHT, which is removed via line 19. Heavies in the dehydrogenation effluent containing, for example, C₂₁+ compounds from polyalkylation reactions occurring in the hydroalkylation unit 12 or methylfluorene byproduct from the dehydrogenation reactions, are collected in line 21 and recovered for use as fuel or as feedstock to other chemical processes.

Although not shown in Figure 1, the second intermediate stream, rich in 2,X'-DMBP isomers and residual MCHT, is supplied by line 19 to a separate isomerization section comprising a hydrogenation unit, then a transalkylation unit and finally a second dehydrogenation unit. As discussed above, the hydrogenation unit converts the 2,X'-DMBP isomers back to the corresponding MCHT isomers, then the transalkylation unit reacts the MCHT (both converted from, and residual in, the second intermediate stream) with toluene to produce a transalkylation product having a different MCHT isomer distribution that the second intermediate stream. The second dehydrogenation unit then converts the MCHT in the transalkylation product to DMBP having a different isomer distribution than that in line 15. In some embodiments, the hydrogenation unit and the transalkylation unit can be combined as a single reactor.

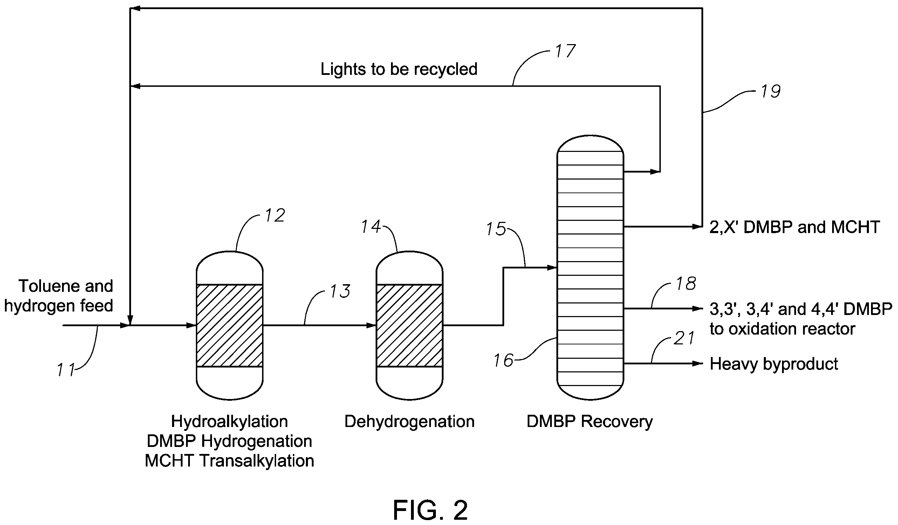

A second embodiment of a process for selectively converting toluene to 3,3'-, 3,4'- and 4,4'-DMBP is shown in Figure 2, in which like numerals indicate like components to the process shown in Figure 1. In the process shown in Figure 2, rather than being supplied to a separate isomerization section, the second intermediate stream, rich in 2,X'-DMBP isomers and residual MCHT, is recycled by line 19 to the hydroalkylation unit 12. In this way, the hydrogenation of the 2,X'-DMBP isomers in the second intermediate stream and transalkylation of the resulting MCHT are conducted in the same reactor as that used to hydroalkylate the toluene feed. To assist this combination of reactions, a transalkylation catalyst can be included in the hydroalkylation unit 12 in addition to the hydroalkylation catalyst.

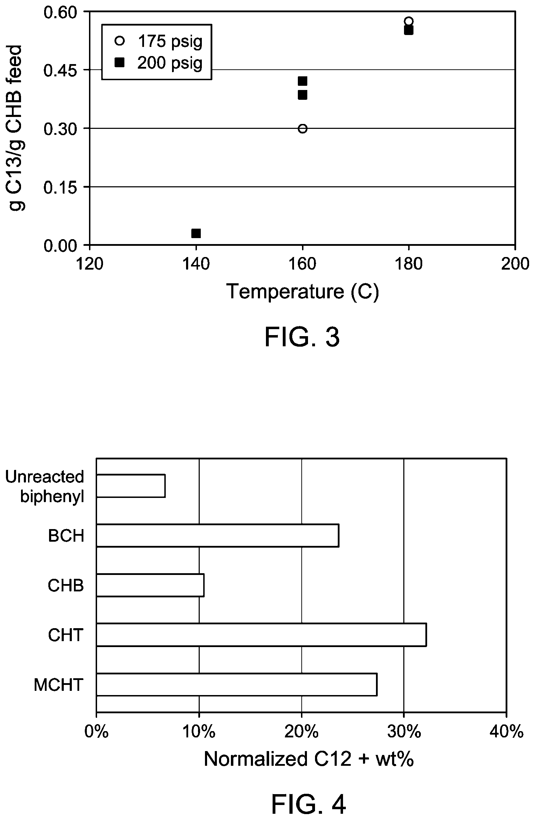

The invention will now be more particularly described with reference to the following non-limiting Examples and Figures 3 and 4 of the accompanying drawings.

This invention further relates to:
1. A process for producing dialkylbiphenyl compounds, the process comprising:
   (a1) providing a feed comprising substituted cyclohexylbenzene isomers having the formula (I): 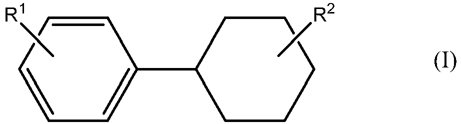 wherein each of R¹ and R² is an alkyl group and wherein the feed comprises m % by weight of isomers in which R¹ is in the 2-position, based on the total weight of substituted cyclohexylbenzene isomers in the feed;
   (a2) reacting the feed under transalkylation conditions with a compound of formula (II): 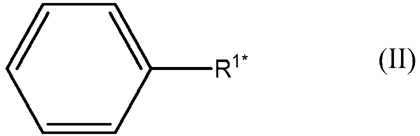 where R^{1*} is an alkyl group, to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
   (a3) dehydrogenating at least part of the transalkylation product under conditions effective to convert at least part of the substituted cyclohexylbenzene isomers in the transalkylation product to dialkylbiphenyl compounds.
2. The process of paragraph 1, wherein each of R^{1*}, R¹ and R² is a methyl group.
3. The process of paragraph 1 or 2, wherein the reacting (a2) is conducted at a temperature of 75 to 250°C.
4. The process of any of paragraphs 1 to 3, wherein the reacting (a2) is conducted in the presence of a solid acid catalyst.
5. The process of any of paragraphs 1 to 4, wherein the reacting (a2) is conducted in the presence of a solid acid catalyst comprising zeolite Y, beta or mordenite.
6. The process of any of paragraphs 1 to 5, wherein m is at least 15 wt% and n is less than 60 wt%.
7. A process for reducing the amount of 2,X' dialkylbiphenyl isomers (where X' is 2', 3' and/or 4') in a first mixture containing the same, the process comprising:
   (b1) hydrogenating the first mixture under conditions effective to produce a hydrogenation product comprising substituted cyclohexylbenzene isomers having the formula (I): 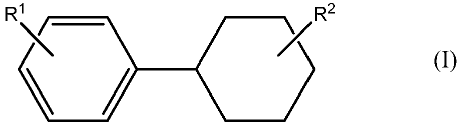 wherein each of R¹ and R² is an alkyl group and wherein the hydrogenation product comprises m % by weight of isomers in which R¹ is in the 2-position, based on the total weight of substituted cyclohexylbenzene isomers in the hydrogenation product;
   (b2) reacting the hydrogenation product under transalkylation conditions with a compound of formula (II): 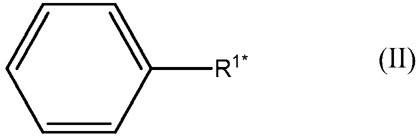 wherein R^{1*} is an alkyl group, to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
   (b3) dehydrogenating at least part of the substituted cyclohexylbenzene isomers in the transalkylation product to produce a product mixture containing less 2,3' and 2,4' dialkylbiphenyl isomers than the first mixture.
8. The process of paragraph 7, wherein each of R^{1*}, R¹ and R² is a methyl group.
9. The process of paragraph 7 or 8, wherein the reacting (b2) is conducted at a temperature of 75 to 250°C.
10. The process of any one of paragraphs 7 to 9, wherein the reacting (b2) is conducted in the presence of a solid acid catalyst.
11. The process of any one of paragraphs 7 to 10, wherein the reacting (b2) is conducted in the presence of a solid acid catalyst comprising zeolite Y, beta or mordenite.
12. The process of any one of paragraphs 7 to 11, wherein m is at least 15 wt% and n is less than 60 wt%.
13. A process for producing dimethylbiphenyl compounds, the process comprising:
   (c1) contacting toluene with hydrogen under hydroalkylation conditions effective to produce a hydroalkylation product comprising (methylcyclohexyl)toluenes;
   (c2) dehydrogenating at least part of the hydroalkylation product under conditions effective to produce a first dehydrogenation product comprising dimethylbiphenyl isomers and unreacted (methylcyclohexyl)toluenes;
   (c3) separating the first dehydrogenation product into a first fraction comprising one or more 3,3', 3,4' and 4,4' dimethylbiphenyl isomers and a second fraction comprising one or more 2,X'-dimethylbiphenyl isomers (where X' is 2, 3, or 4) and at least part of the unreacted (methylcyclohexyl)toluenes;
   (c4) hydrogenating at least part of the dimethylbiphenyl isomers in the second fraction to the corresponding (methylcyclohexyl)toluene isomers and produce a hydrogenation effluent;
   (c5) reacting at least part of the hydrogenation effluent from (c4) with toluene under transalkylation conditions affective to produce a transalkylation product containing less material represented by Formula (X) than the hydrogenation effluent, where Formula (X) is 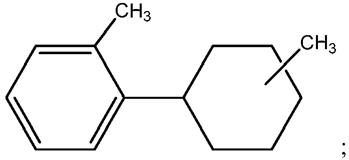 and
   (c6) dehydrogenating at least part of the transalkylation product to produce a second dehydrogenation product comprising less 2,X'-dimethylbiphenyl isomers than the first dehydrogenation product.
14. The process of paragraph 13, wherein the contacting (c1) and the reacting (c5) are conducted in sequential reaction zones.
15. The process of paragraph 13, wherein the contacting (c1) and the reacting (c5) are conducted in the same reaction zone.
16. The process of any one of paragraphs 13 to 15, wherein the hydrogenating (c4) and the reacting (c5) are conducted in sequential reaction zones.
17. The process of any one of paragraphs 13 to 15, wherein the hydrogenating (c4) and the reacting (c5) are conducted in the same reaction zone.
18. The process of paragraph 13, wherein the contacting (c1), the hydrogenating (c4) and the reacting (c5) are conducted in the same reaction zone.
19. The process of paragraph 18, wherein the reaction zone contains a catalyst composition comprising a molecular sieve of the MCM-22 family, a FAU-type molecular sieve and a hydrogenation/dehydrogenation component.
20. The process of any preceding paragraph, wherein m is at least 5 wt% and n is less than 75 wt%.
21. The process of any of paragraphs 1 to 20, wherein the catalyst used for hydrogenation in step (b1) comprises a transition metal.
22. The process of paragraph 21, wherein the metal is a Group 3, 4, 5, 6, 7, 8, 9, 10, 11, or 12 metal.
23. The process of paragraph 22, wherein the metal is Pt or Pd.

### Example 1

The experiments described in Examples 1 and 2 utilized a reactor unit with 8 parallel reactors heated by furnace. For different tests anywhere from 1-8 reactors could be utilized. The reactors used in these experiments consisted of quartz tubes of 9 mm in diameter. Annular N₂ flow on the outside of the quartz reactor allowed for pressure equilibration between the inside and outside of each reactor channel. Catalyst extrudates were crushed to 20/40 mesh loaded in quantities ranging from 0.25-2g (to vary corresponding weight based space velocity) after being diluted up to 4g in crushed quartz. A quartz wool plug was used at the top and bottom of the catalyst bed to keep catalyst in place. Two sets of 4 parallel reactors were placed in heated furnaces to control isothermal reaction temperature. Each reactor contained an internal thermocouple in the catalyst bed in a 1/8" (3.175 mm) thermowell. The reactors were topped off with the same quartz chips.

The catalysts in all reactors were pre-conditioned *in situ* as described in the subsequent Examples. An ISCO syringe pump was used to introduce the feed to the reactor. The feed was pumped through a vaporizer before being mixed in-line with H₂ and/or N₂ at a molar ratio of between 0 and 2 (gas to hydrocarbon liquid). The products exiting the reactor were condensed and collected in intervals (1-2 samples per day per reactor) and analyzed offline by GC or GCMS.

GC analysis was conducted on an Agilent 7890 GC equipped with FID detector and an Automatic liquid sampler (ALS). Typical injection size was about 0.2µl. The columns used were from Supelco of the Dex type. A Gamma DEX column was joined together with a Beta Dex column to give a total length of 120m (60m for each type). The ID of the columns was 0.25mm. The GC was operated in constant flow mode with an I\initial pressure of about 78psi (538 kPa) and column flow of about 3.0ml/min using helium as carrier gas. The following oven procedure was employed:
- Initial temperature of 140°C, hold for 30 minutes
- Ramp 1 at 2°C/min to 180°C, hold for 20 minutes
- Ramp 2 at 3°C/min to 220°C, hold for 7 minutes
- Total analysis time of 90.33 minutes.

A similar method was used for the GC-MS measurements. The instrument was an Agilent 7890 GC equipped with a 5975C MSD detector and FID. The main difference is that this setup had a purged 2-way splitter that enabled a sample to be simultaneously analyzed on two detectors using a single injection. The injection size was about 0.5µl. Additionally, an auxiliary helium pressure of 6 psi (41 kPa) was used for the purged splitter. The final oven time was extended by 20 minutes for a total analysis time of 110 minutes.

In Example 1, one reactor of the reactor unit described above was utilized to study the transalkylation of cyclohexylbenzene (CHB) with toluene. 1 gram of a commercial USY catalyst was loaded into the reactor channel and the catalyst was dried in N₂ overnight at 260°C. A liquid feed consisting of 33% cyclohexylbenzene and 67% toluene by weight was fed to the reactor at 1 WHSV with a co-feed of nitrogen at a 0.7:1 nitrogen to hydrocarbon molar ratio.

The results are shown in Figure 3 and Table 1 below.

**Table 1**

| | | | | |
|---|---|---|---|---|
| Temperature (°C) | 180 | 160 | 160 | 180 |
| Pressure (psig (kPa)) | 200 (1379) | 200 (1379) | 175 (1207) | 175 (1207) |
| Cyclohexylbenzene (CHB) conversion | 87% | 44% | 28% | 86% |
| Moles C₁₃H₁₈/Moles CHB consumed | 59% | 88% | 100% | 62% |

In Example 1, CHB is used as a surrogate for MCHT to test the viability of transalkylation to change the isomer distribution of MCHT. Figure 3 shows the yield of C₁₃ products (g produced per g CHB fed) as a function of temperature and pressure. The rates at 140°C are low, but significant. The yield increases with temperature as is seen in Table 1 and Figure 3. These results prove the concept that MCHT transalkylation with toluene can be used to effectively change the methyl group position on the aromatic ring of the molecules. Undesired products include CHB decomposition to benzene and alkanes as well as xylene formation and the formation of heavier species. In these experiments, the xylene yield was less than 0.001g/g in all cases. This means that transalkylation of toluene at the benzylic carbon of CHB is much more favorable than methyl disproportionation.

### Example 2

The procedure and apparatus of Example 1 were employed but with one reactor channel being utilized to study the sequential hydrogenation and transalkylation of biphenyl in a stacked bed configuration. 1 gram each of a commercial platinum/palladium hydrogenation catalyst and a commercial USY catalyst were loaded in a stacked bed configuration with the hydrogenation catalyst upstream of the zeolite catalyst. The catalysts were dried in N₂ overnight at 260°C and then a reduction procedure followed where 5 sccm H₂ was flowed over the bed at 50 psig (345 kPa) and the temperature was ramped at 5°C/min to 290°C. This temperature was held for 3 hours and then the reactor was cooled to the starting reaction temperature of 200°C. A liquid feed consisting of 10% biphenyl and 90% toluene by weight was fed to the reactor at 1 WHSV (total) with a co-feed of hydrogen at a 2:1 hydrogen to hydrocarbon molar ratio.

In this example, at 200°C and 200 psig (1480 kPa-absolute), greater than 90% of the biphenyl in the feed was converted. The major C₁₂ or greater products were cyclohexylbenzene (CHB) and bicyclohexane (BCH) from hydrogenation of the biphenyl, cyclohexyltoluene (CHT, C₁₃H₁₈) from the desired sequence of hydrogenation and transalkylation, and methylcyclohexyltoluene (MCHT) which was likely a result of toluene hydroalkylation over the multiple catalyst functions. The normalized distribution of these products in the reactor effluent liquid is shown in Figure 5.

### Example 3

In this Example, a study was conducted on the sequential hydrogenation and toluene transalkylation of dimethylbiphenyl in a mixed bed configuration with simultaneous toluene transalkylation. The reaction test was carried out in a fixed bed down-flow reactor having a diameter of 0.5 inch (1.3 cm). The catalysts used in the study were a mixed bed of commercial USY/Al₂O₃ and Pd/MCM-49 hydroalkylation catalyst (in varying ratio). The catalyst charge was 4 g. The catalyst was activated by heating up the catalyst bed to 260°C under nitrogen and drying overnight. The catalyst was then reduced in hydrogen at 290°C (ramp rate of 5°C/min) for 3 hours. Hydrogen flow rate was 50 sccm. Reactor pressure was maintained constant, at 50 psig (346 kPa-absolute), throughout catalyst activation.

The liquid feed used in this test was a mixture of 90% anhydrous toluene and 10% 2,3' DMBP. Hydrogen to hydrocarbon ratio of 2:1 (molar) was used. The total WHSV used in this experiment was 1, the reaction pressure was 175 psig (1308 kPa-absolute) and the temperature was 165°C or 185°C. The results are shown in Table 2 below:

**Table 2**

| | | | |
|---|---|---|---|
| Ratio USY:Pd/MCM-49 | 1 | 1 | 3 |
| Time on stream (days) | 3 | 6 | 3 |
| Temperature (°C) | 165 | 185 | 165 |
| Pressure (psig (kPa)) | 175 (1207) | 175 (1207) | 175 (1207) |
| 2,3' DMBP conversion | 38% | 57% | 53% |
| Toluene conversion | 47% | 24% | 28% |
| g 2,3' DMBP/ g product | 6% | 4% | 5% |
| g 2,X' MCHT/ g product | 5% | 4% | 5% |
| g bimethylcyclohexane (BCMH)/ g product | 2% | 1% | 1% |

As is seen in Table 2, this configuration allows for the consumption of 2,3' DMBP with simultaneous hydroalkylation. It also shows the viability of consuming 2,X' DMBP and 2,X' MCHT at the same rate as 2,X' MCHT is being produced by the hydroalkylation reaction. It should be noted that direct isomerization of the 2,3' DMBP is also a possible reaction pathway for consumption in addition to the sequential hydrogenation and transalkylation described here.

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

## Claims

1. A process for producing dialkylbiphenyl compounds, the process comprising:
(a1) providing a feed comprising substituted cyclohexylbenzene isomers having the formula (I): 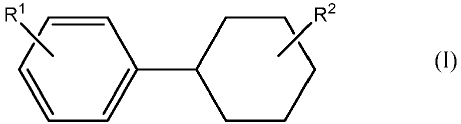 wherein each of R¹ and R² is an alkyl group and wherein the feed comprises m % by weight of isomers in which R¹ is in the 2-position, based on the total weight of substituted cyclohexylbenzene isomers in the feed;
(a2) reacting the feed under transalkylation conditions with a compound of formula (II): 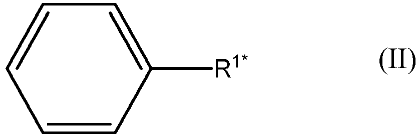
where R^{1*} is an alkyl group,
to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
(a3) dehydrogenating at least part of the transalkylation product under conditions effective to convert at least part of the substituted cyclohexylbenzene isomers in the transalkylation product to dialkylbiphenyl compounds.

2. The process of claim 1, wherein each of R^{1*}, R¹ and R² is a methyl group.

3. The process of claim 1 or 2, wherein the reacting (a2) is conducted at a temperature of 75 to 250°C.

4. The process of any of claims 1 to 3, wherein the reacting (a2) is conducted in the presence of a solid acid catalyst.

5. The process of any of claims 1 to 4, wherein the reacting (a2) is conducted in the presence of a solid acid catalyst comprising zeolite Y, beta or mordenite.

6. The process of any of claims 1 to 5, wherein m is at least 15 wt% and n is less than 60 wt%.

7. A process for reducing the amount of 2,X' dialkylbiphenyl isomers (where X' is 2', 3', and/or 4') in a first mixture containing the same, the process comprising:
(b1) hydrogenating the first mixture under conditions effective to produce a hydrogenation product comprising substituted cyclohexylbenzene isomers having the formula (I): 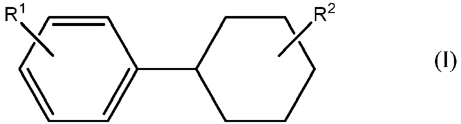 wherein each of R¹ and R² is an alkyl group and wherein the hydrogenation product comprises m % by weight of isomers in which R¹ is in the 2-position, based on the total weight of substituted cyclohexylbenzene isomers in the hydrogenation product;
(b2) reacting the hydrogenation product under transalkylation conditions with a compound of formula (II): 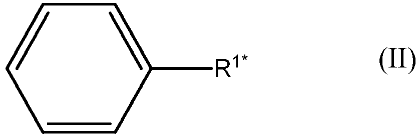
where R^{1*} is an alkyl group,
to produce a transalkylation product comprising substituted cyclohexylbenzene isomers having the formula (I) and including n % by weight of isomers in which R¹ is in the 2-position, based on the total weight of substituted cyclohexylbenzene isomers in the transalkylation product, wherein n<m; and
(b3) dehydrogenating at least part of the substituted cyclohexylbenzene isomers in the transalkylation product to produce a product mixture containing less 2,3' and 2,4' dialkylbiphenyl isomers than the first mixture.

8. The process of claim 7, wherein each of R^{1*}, R¹ and R² is a methyl group.

9. The process of claim 7 or 8, wherein the reacting (b2) is conducted at a temperature of 75 to 250°C.

10. The process of any of claims 7 to 9, wherein the reacting (b2) is conducted in the presence of a solid acid catalyst.

11. The process of any of claims 7 to 10, wherein the reacting (b2) is conducted in the presence of a solid acid catalyst comprising zeolite Y, beta or mordenite.

12. The process of any of claims 7 to 11, wherein m is at least 15 wt% and n is less than 60 wt%.

13. A process for producing dimethylbiphenyl compounds, the process comprising:
(c1) contacting toluene with hydrogen under hydroalkylation conditions effective to produce a hydroalkylation product comprising (methylcyclohexyl)toluenes;
(c2) dehydrogenating at least part of the hydroalkylation product under conditions effective to produce a first dehydrogenation product comprising dimethylbiphenyl isomers and unreacted (methylcyclohexyl)toluenes;
(c3) separating the first dehydrogenation product into a first fraction comprising one or more 3,3', 3,4' and 4,4' dimethylbiphenyl isomers and a second fraction comprising one or more 2,X'-dimethylbiphenyl isomers (where X' is 2, 3 or 4) and at least part of the unreacted (methylcyclohexyl)toluenes;
(c4) hydrogenating at least part of the dimethylbiphenyl isomers in the second fraction to the corresponding (methylcyclohexyl)toluene isomers and produce a hydrogenation effluent;
(c5) reacting at least part of the hydrogenation effluent from (c4) with toluene under transalkylation conditions effective to produce a transalkylation product containing less material represented by Formula (X) than the hydrogenation effluent, where Formula (X) is 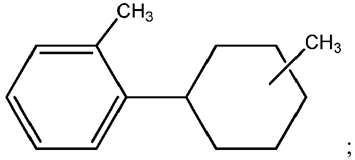 and
(c6) dehydrogenating at least part of the transalkylation product to produce a second dehydrogenation product comprising less 2,X'-dimethylbiphenyl isomers than the first dehydrogenation product.

14. The process of claim 13, wherein the contacting (c1), the hydrogenating (c4) and the reacting (c5) are conducted in the same reaction zone.

15. The process of claim 14, wherein the reaction zone contains a catalyst composition comprising a molecular sieve of the MCM-22 family, a FAU-type molecular sieve and a hydrogenation/dehydrogenation component.

## Patentansprüche

1. Verfahren zur Herstellung von Dialkylbiphenylverbindungen, wobei das Verfahren umfasst:
(a1) Bereitstellen eines Einsatzmaterials, das substituierte Cyclohexylbenzolisomere mit der Formel (1) umfasst: 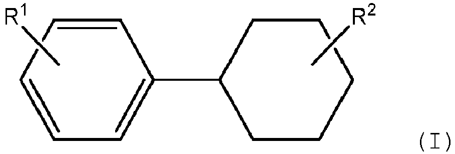 in der jedes von R¹ und R² eine Alkylgruppe ist und wobei das Einsatzmaterial m Gew.-% Isomere umfasst, in denen R¹ in der 2-Position vorliegt, bezogen auf das Gesamtgewicht der substituierten Cyclohexylbenzolisomere in dem Einsatzmaterial,
(a2) Umsetzen des Einsatzmaterials unter Transalkylierungsbedingungen mit einer Verbindung der Formel (II): 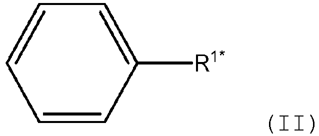
in der R^{1*} eine Alkylgruppe ist,
um ein Transalkylierungsprodukt herzustellen, das substituierte Cyclohexylbenzolisomere mit der Formel (I) umfasst und n Gew.-% Isomere umfasst, in denen R¹ in der 2-Position vorliegt, bezogen auf das Gesamtgewicht der substituierten Cyclohexylbenzolisomere in dem Transalkylierungsprodukt, wobei n < m ist, und
(a3) Dehydrieren mindestens eines Teils des Transalkylierungsprodukts unter Bedingungen, die wirksam sind, mindestens einen Teil der substituierten Cyclohexyl-benzolisomere in dem Transalkylierungsprodukt in Dialkylbiphenylverbindungen umzuwandeln.

2. Verfahren nach Anspruch 1, bei dem jedes von R^{1*}, R¹ und R² eine Methylgruppe ist.

3. Verfahren nach Anspruch 1 oder 2, bei dem das Umsetzen (a2) bei einer Temperatur von 75 bis 250°C durchgeführt wird.

4. Verfahren nach einem der Ansprüche 1 bis 3, bei dem das Umsetzen (a2) in Gegenwart eines festen Säurekatalysators durchgeführt wird.

5. Verfahren nach einem der Ansprüche 1 bis 4, bei dem das Umsetzen (a2) in Gegenwart eines festen Säurekatalysators durchgeführt wird, der Zeolith Y, Beta oder Mordenit umfasst.

6. Verfahren nach einem der Ansprüche 1 bis 5, bei dem m mindestens 15 Gew.-% ist und n weniger als 60 Gew.-% ist.

7. Verfahren zur Verringerung der Menge an 2,X'-Dialkylbiphenylisomeren (wobei X' 2', 3', und/oder 4' ist) in einer ersten Mischung, die diese enthält, wobei das Verfahren umfasst:
(b1) Hydrieren der ersten Mischung unter Bedingungen, die wirksam sind, ein Hydrierungsprodukt zu erzeugen, das substituierte Cyclohexylbenzolisomere mit der Formel (I) umfasst: 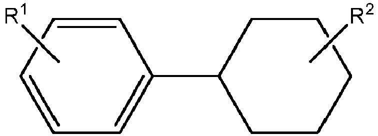 in der jedes von R¹ und R² eine Alkylgruppe ist und wobei das Hydrierungsprodukt m Gew.-% Isomere umfasst, in denen R¹ in der 2-Position vorliegt, bezogen auf das Gesamtgewicht der substituierten Cyclohexylbenzolisomere in dem Hydrierungsprodukt,
(b2) Umsetzen des Hydrierungsprodukts unter Transalkylierungsbedingungen mit einer Verbindung der Formel (II): 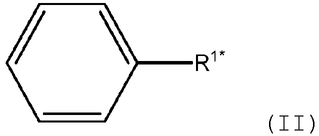
in der R^{1*} eine Alkylgruppe ist,
um ein Transalkylierungsprodukt herzustellen, das substituierte Cyclohexylbenzolisomere mit der Formel (I) umfasst und n Gew.-% Isomere umfasst, in denen R¹ in der 2-Position vorliegt, bezogen auf das Gesamtgewicht der substituierten Cyclohexylbenzolisomere in dem Transalkylierungsprodukt, wobei n < m ist, und
(b3) Dehydrieren mindestens eines Teils der substituierten Cyclohexylbenzolisomere in dem Transalkylierungsprodukt, um eine Produktmischung herzustellen, die weniger 2,3'- und 2,4'-Dialkylbiphenylisomere enthält als die erste Mischung.

8. Verfahren nach Anspruch 7, bei dem jedes von R^{1*}, R¹ und R² eine Methylgruppe ist.

9. Verfahren nach Anspruch 7 oder 8, bei dem das Umsetzen (b2) bei einer Temperatur von 75 bis 250°C durchgeführt wird.

10. Verfahren nach einem der Ansprüche 7 bis 9, bei dem das Umsetzen (b2) in Gegenwart eines festen Säurekatalysators durchgeführt wird.

11. Verfahren nach einem der Ansprüche 7 bis 10, bei dem das Umsetzen (b2) in Gegenwart eines festen Säurekatalysators durchgeführt wird, der Zeolith Y, Beta oder Mordenit umfasst.

12. Verfahren nach einem der Ansprüche 7 bis 11, bei dem m mindestens 15 Gew.-% ist und n weniger als 60 Gew.-% ist.

13. Verfahren zur Herstellung von Dimethylbiphenylverbindungen, wobei das Verfahren umfasst:
(c1) In-Kontakt-Bringen von Toluol mit Wasserstoff unter Hydroalkylierungsbedingungen, die wirksam sind, ein Hydroalkylierungsprodukt zu erzeugen, das (Methylcyclohexyl)toluole umfasst,
(c2) Dehydrieren mindestens eines Teils des Hydroalkylierungsprodukts unter Bedingungen, die wirksam sind, ein erstes Dehydrierungsprodukt zu erzeugen, das Dimethylbiphenylisomere und nicht-umgesetzte (Methyl-cyclohexyl)toluole umfasst,
(c3) Trennen des ersten Dehydrierungsprodukts in eine erste Fraktion, die ein oder mehrere 3,3'-, 3,4'- und 4,4'-Dimethylbiphenylisomere umfasst, und eine zweite Fraktion, die ein oder mehrere 2,X'-Dimethylbiphenylisomere (in denen X' 2, 3 oder 4 ist) und nicht-umgesetzte (Methylcyclohexyl)toluole umfasst,
(c4) Hydrieren mindestens eines Teils der Dimethylbiphenylisonmere in der zweiten Fraktion zu den entsprechenden (Methylcyclohexyl)toluolisomeren und Erzeugen eines Hydrierungsaustrittsmaterials,
(c5) Umsetzen mindestens eines Teils des Hydrierungsaustrittsmaterials aus (c4) mit Toluol unter Transalkylierungsbedingungen, die wirksam sind, ein Transalkylierungsprodukt zu erzeugen, das weniger Material enthält, das durch die Formel (X) dargestellt ist, als das Hydrierungsaustrittsmaterial, wobei die Formel (X): 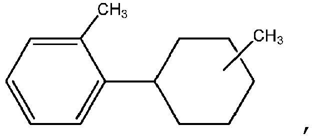 und
(c6) Dehydrieren mindestens eines Teils des Transalkylierungsprodukts, um ein zweites Dehydrierungsprodukt zu erzeugen, das weniger 2,X'-Dimethylbiphenylisomere umfasst als das erste Dehydrierungsprodukt.

14. Verfahren nach Anspruch 13, bei dem das In-Kontakt-Bringen (c1), das Hydrieren (c4) und das Umsetzen (c5) in der gleichen Reaktionszone durchgeführt werden.

15. Verfahren nach Anspruch 14, bei dem die Reaktionszone eine Katalysatorzusammensetzung enthält, die Molekularsieb der MCM-22-Familie, FAU-Typ-Molekularsieb und eine Hydrierungs-/Dehydrierungskomponente umfasst.

## Revendications

1. Procédé de production de composés de dialkylbiphényle, le procédé comprenant :
(a1) la fourniture d'une matière première comprenant des isomères de cyclohexylbenzène substitué ayant la formule (I) : 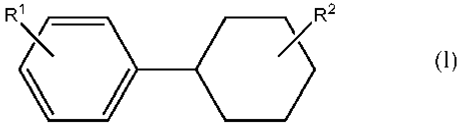 dans lequel chacun de R¹ et R² est un groupe alkyle et dans lequel la matière première comprend m % en poids d'isomères dans lesquels R¹ est à la position 2, sur la base du poids total d'isomères de cyclohexylbenzène substitué dans la matière première ;
(a2) réaction de la matière première dans des conditions de transalkylation avec un composé de formule (II) : 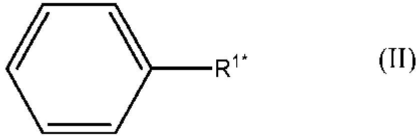
où R^{1*} est un groupe alkyle,
pour produire un produit de transalkylation comprenant des isomères de cyclohexylbenzène substitué ayant la formule (I) et comprenant n % en poids d'isomères dans lesquels R¹ est à la position 2, sur la base du poids total d'isomères de cyclohexylbenzène substitué dans le produit de transalkylation, dans lequel n < m ; et
(a3) déshydrogénation d'au moins une partie du produit de transalkylation dans des conditions efficaces pour convertir au moins une partie des isomères de cyclohexylbenzène substitué dans le produit de transalkylation en composés de dialkylbiphényle.

2. Procédé selon la revendication 1, dans lequel chacun de R¹*, R¹ et R² est un groupe méthyle.

3. Procédé selon la revendication 1 ou 2, dans lequel la réaction (a2) est conduite à une température de 75 à 250 °C.

4. Procédé selon l'une quelconque des revendications 1 à 3, dans lequel la réaction (a2) est conduite en présence d'un catalyseur acide solide.

5. Procédé selon l'une quelconque des revendications 1 à 4, dans lequel la réaction (a2) est conduite en présence d'un catalyseur acide solide comprenant la zéolite Y, bêta ou mordénite.

6. Procédé selon l'une quelconque des revendications 1 à 5, dans lequel m est au moins 15 % en poids et n est inférieur à 60 % en poids.

7. Procédé de réduction de la quantité d'isomères de 2,X'-dialkylbiphényle (où X' est 2', 3 et/ou 4') dans un premier mélange contenant ceux-ci, le procédé comprenant :
(b1) l'hydrogénation du premier mélange dans des conditions efficaces pour produire un produit d'hydrogénation comprenant des isomères de cyclohexylbenzène substitué ayant la formule (I) : 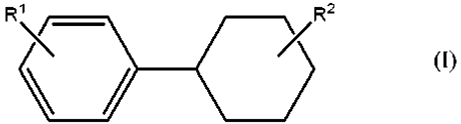 dans lequel chacun de R¹ et R² est un groupe alkyle et dans lequel le produit d'hydrogénation comprend m % en poids d'isomères dans lequel R¹ est à la position 2, sur la base du poids total d'isomères de cyclohexylbenzène substitué dans le produit d'hydrogénation ;
(b2) la réaction du produit d'hydrogénation dans des conditions de transalkylation avec un composé de formule (II) : 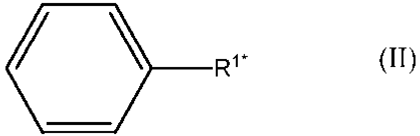
où R^{1*} est un groupe alkyle,
pour produire un produit de transalkylation comprenant des isomères de cyclohexylbenzène substitué ayant la formule (1) et comprenant n % en poids d'isomères dans lesquels R¹ est à la position 2, sur la base du poids total d'isomères de cyclohexylbenzène substitué dans le produit de transalkylation, dans lequel n < m ; et
(b3) la déshydrogénation d'au moins une partie des isomères de cyclohexylbenzène substitué dans le produit de transalkylation pour produire un mélange de produit contenant moins d'isomères 2,3' et 2,4'-dialkylbiphényle que le premier mélange.

8. Procédé selon la revendication 7, dans lequel chacun de R¹*, R¹ et R² est un groupe méthyle.

9. Procédé selon la revendication 7 ou 8, dans lequel la réaction (b2) est conduite à une température de 75 à 250 °C.

10. Procédé selon l'une quelconque des revendications 7 à 9, dans lequel la réaction (b2) est conduite en présence d'un catalyseur acide solide.

11. Procédé selon l'une quelconque des revendications 7 à 10, dans lequel la réaction (b2) est conduite en présence d'un catalyseur acide solide comprenant la zéolite Y, bêta ou mordénite.

12. Procédé selon l'une quelconque des revendications 7 à 11, dans lequel m est au moins 15 % en poids et n est inférieur à 60 % en poids.

13. Procédé de production de composés de diméthylbiphényle, le procédé comprenant :
(c1) la mise en contact de toluène avec de l'hydrogène dans des conditions d'hydroalkylation efficaces pour produire un produit d'hydroalkylation comprenant des (méthylcyclohexyl)toluènes ;
(c2) la déshydrogénation d'au moins une partie du produit d'hydroalkylation dans des conditions efficaces pour produire un premier produit de déshydrogénation comprenant des isomères de diméthylbiphényle et des (méthylcyclohexyl)toluènes n'ayant pas réagi ;
(c3) la séparation du premier produit de déshydrogénation dans une première fraction comprenant un ou plusieurs isomères 3,3', 3,4' et 4,4'-diméthylbiphényle et une deuxième fraction comprenant un ou plusieurs 2,X'-isomères de diméthylbiphényle (où X' est 2, 3 ou 4) et au moins une partie des (méthylcyclohexyl)toluènes n'ayant pas réagi ;
(c4) l'hydrogénation d'au moins une partie des isomères de diméthylbiphényle dans la deuxième fraction en isomères de (méthylcyclohexyl)toluène correspondants et la production d'un effluent d'hydrogénation ;
(c5) la réaction d'au moins une partie de l'effluent d'hydrogénation de (c4) avec du toluène dans des conditions de transalkylation efficaces pour produire un produit de transalkylation contenant moins de matériau représenté par la formule (X) que l'effluent d'hydrogénation, où la formule (X) est 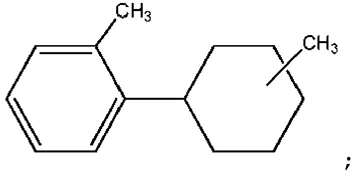 et
(c6) la déshydrogénation d'au moins une partie du produit de transalkylation pour produire un deuxième produit de déshydrogénation comprenant moins d'isomères 2,X'-diméthylbiphényle que le produit de déshydrogénation.

14. Procédé selon la revendication 13, dans lequel la mise en contact (c1), l'hydrogénation (c4) et la réaction (c5) sont conduits dans la même zone de réaction.

15. Procédé selon la revendication 14, dans lequel la zone de réaction contient une composition de catalyseur comprenant un tamis moléculaire de la famille MCM-22, un tamis moléculaire de type FAU et un composant d'hydrogénation/déshydrogénation.
